# FASCICULE DE BREVET EUROPEEN

(11) **EP 0 891 959 B1**
(45) Date de publication et mention de la délivrance du brevet: **21.11.2001**
(21) Numéro de dépôt: 98401664.2
(22) Date de dépôt: 03.07.1998
(51) Int. Cl.: C07C 17/16, C07B 39/00, C07C 41/22

(54) **Procédé de préparation des chlorures aliphatiques ou cycloaliphatiques**
Verfahren zur Herstellung von aliphatischen oder cyclaliphatischen Chloriden
Process for the preparation of aliphatic or cycloaliphatic chlorides

(30) Priorité: 16.07.1997 FR 9709031
(43) Date de publication de la demande: 20.01.1999
(73) Titulaire: SOCIETE NATIONALE DES POUDRES ET EXPLOSIFS, F-75181 Paris Cédex 04 (FR)
(72) Inventeur: Lecomte, Loic, 31400 Toulouse (FR); Metge, Serge, 31300 Toulouse (FR); Souyri, Denis, 31400 Toulouse (FR)

(56) Documents cités:
- EP-A- 0 213 976
- EP-A- 0 545 774
- EP-A- 0 645 357
- US-A- 2 926 204
- DATABASE WPI Section Ch, Derwent Publications Ltd., London, GB; Class E16, AN 71-44440S XP002059004 & SU 277 774 A (PERM BRANCH APPLIED CHEM)

## Description

La présente invention concerne un nouveau procédé de préparation des chlorures d'hydrocarbures aliphatiques ou cycloaliphatiques par réaction de l'alcool correspondant avec le phosgène ou le chlorure de thionyle en présence d'un sel de guanidinium hexasubstitué comme catalyseur.

Les chlorures d'hydrocarbures aliphatiques ou cycloaliphatiques sont des composés connus et très utiles en tant que tels ou comme intermédiaires de synthèses organiques, notamment dans le domaine des polymères ou dans celui de la protection des plantes.

Les chlorures aliphatiques peuvent être préparés à partir de l'alcool correspondant en les faisant réagir avec l'acide chlorhydrique, mais la réaction est réversible et la transformation n'est pas complète. Sa mise en oeuvre est longue et délicate. Il faut souvent opérer sous une pression supérieure à la pression atmosphérique. Des composés isomères et d'autres produits secondaires se forment ainsi qu'une phase aqueuse qui est gênante. De plus, ce procédé ne permet pas de transformer convenablement des alcools comportant des fonctions très sensibles à l'acide chlorhydrique et en particulier les alcools insaturés.

Quelques procédés dans lesquels on fait réagir un alcool avec du phosgène en présence d'un catalyseur ont été décrits.

Dans la demande de brevet EP 645 357, la réaction de l'alcool avec le phosgène est effectuée en présence de catalyseurs qui sont des composés d'addition de formamides N,N-disubstitués avec du phosgène, mais ces dérivés de formamides se dégradent à température élevée et donnent naissance à de nombreux sous-produits.

Selon le procédé de la demande de brevet EP 514 683, on fait réagir l'alcool avec du phosgène ou du chlorure de thionyle en présence d'oxydes de phosphine comme catalyseurs. La réaction est très longue et des impuretés phosphorées se forment.

Selon d'autres procédés, on forme tout d'abord les chloroformiates que l'on décompose ensuite en présence de catalyseurs tels que des sels d'ammonium ou de phosphonium quaternaires comme décrits dans le brevet US 4 734 535. Mais certains chloroformiates, comme par exemple le chloroformiate de propargyle, sont très difficiles à préparer. Les chloroformiates sont de plus très réactifs vis à vis des alcools qui sont utilisés pour les préparer. Des sous-produits tels que des carbonates se forment en quantités importantes. Il faut ensuite les séparer. Certains de ces carbonates sont dangereux à la température de réaction utilisée. Les ammoniums quaternaires utilisés comme catalyseurs perdent leur activité au cours de la réaction et ne peuvent pas être réutilisés.

L'invention a pour objet un procédé de préparation des chlorures d'hydrocarbures aliphatiques ou cycloaliphatiques, à partir des alcools correspondants, qui ne présente pas les inconvénients précités.

Selon le procédé de l'invention, on prépare les chlorures d'hydrocarbures aliphatiques ou cycloaliphatiques, saturés ou non, substitués ou non, par réaction du mono- ou du polyalcool correspondant avec le phosgène ou le chlorure de thionyle, en présence d'au moins un catalyseur choisi dans le groupe constitué par:
- (1) les halogénures de guanidinium hexasubstitués et leurs halogénohydrates, de formule (II) : 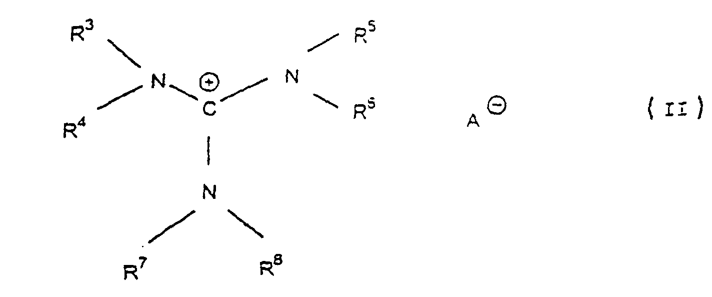 dans laquelle les radicaux R³ à R⁸, identiques ou différents, représentent des groupes alkyles en C₁ à C₁₂, linéaires ou ramifiés, substitués ou non, et/ou cycloalkyles en C₅ à C₆, substitués ou non, et A représente un atome de chlore ou de brome ou un groupe HCl₂ ou HBr₂, et
- (2) les halogénures de guanidinium hexasubstitués et leurs halogénohydrates, greffés par l'intermédiaire d'un de leurs radicaux sur un support à base de silice, de formule (III) : 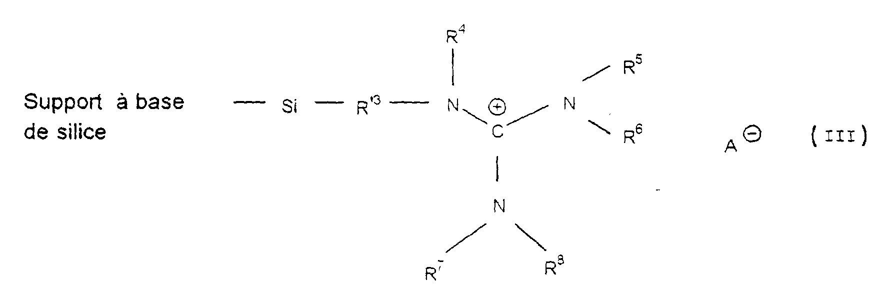 dans laquelle les radicaux R⁴ à R⁸ et l'anion A ont les significations précédentes et le radical R'³ représente un groupe alkylène en C₂ à C₁₀.

Les alcools utilisés comme matières de départ sont préférentiellement, les mono- ou polyalcools de formule (I) : 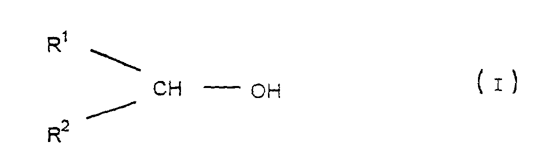 dans laquelle R¹ et R², identiques ou différents, représentent un atome d'hydrogène, un groupe aliphatique en C₁ à C₃₀, de préférence en C₁ à C₂₂, linéaire ou ramifié, substitué ou non, saturé ou non, un groupe cycloaliphatique en C₃ à C₁₂, de préférence en C₃ à C₈, substitué ou non, saturé ou non, un groupe alcoxy en C₁ à C₃₀, substitué ou non, saturé ou non, ou un groupe aryle ou aryloxy, substitué ou non;
ou bien dans laquelle R¹ et R² forment ensemble et avec le carbone sur lequel ils sont fixés, un groupe cycloaliphatique en C₃ à C₁₂, substitué ou non, saturé ou non.

Les substituants de R¹ et R² peuvent être choisis parmi les atomes d'halogène, en particulier de fluor, de chlore et de brome, les groupes hydroxyles, aliphatiques saturés ou non, substitués ou non, halogénoaliphatiques, en particulier le groupe trifluorométhyle, cycloaliphatiques saturés ou non, substitués ou non, aryles substitués ou non, araliphatiques saturés ou non, substitués ou non, alcoxy, polyalcoxy, alcoxyaryles, aryloxy, nitro et cyano. Ces substituants, lorsqu'ils sont des groupes hydrocarbonés peuvent eux-mêmes être substitués par ces différents substituants.

Les liaisons insaturées des chaînes aliphatiques contenues dans R¹ et R² peuvent être des doubles et/ou des triples liaisons.

Les alcools préférés sont les alcools dans lesquels R¹ et R², identiques ou différents, représentent un atome d'hydrogène ou un groupe, substitué ou non, suivant :
- alkyle en C₁ à C₂₂, particulièrement en C₁ à C₈;
- alkényle en C₂ à C₂₂, particulièrement en C₂ à C₈, tel que vinyle, allyle;
- alkinyle en C₂ à C₂₂, particulièrement en C₂ à C₈ et notamment éthynyle;
- alcoxy en C₁ à C₂₂, particulièrement en C₁ à C₄ tel que éthyloxy, propoxy;
- aryle, tel que phényle et naphtyle;
- aryloxy, en particulier phénoxy;
- hydroxyalkyle ou polyhydroxyalkyle en C₁ à C₂₂;
- hydroxyalkényle ou hydroxyalkinyle en C₂ à C₂₂;
- polyhydroxy-alkényle ou -alkinyle en C₂ à C₂₂;
- cycloalkyle en C₃ à C₁₂, particulièrement en C₃ à C₈;
ou les alcools dans lesquels R¹ et R² forment ensemble et avec le carbone sur lequel ils sont fixés, un groupe cycloalkyle ou cycloalkényle en C₃ à C₈.

Les substituants de ces groupes sont de préférence choisis parmi les atomes d'halogène, en particulier de fluor, de chlore et de brome, les groupes alkyle en C₁ à C₄, halogénoalkyle en C₁ à C₄, en particulier CF₃, cycloalkyle en C₃ à C₈, alcoxy en C₁ à C₄, aralkyle en C₇ à C₁₂, aryle, en particulier phényle, C₁-C₂₂ alcoxyphényle, en particulier le 4-méthoxyphényle, aryloxy, en particulier phényloxy.

Comme exemples d'alcools, on peut citer le butanol-1, l'hexanol-1, l'octanol-1, le dodécanol-1, l'hexadécanol-1, le docosanol, le butane-diol-1,4, le butyne-2-diol-1,4, l'hexanediol-1,6, l'octanediol-1,8, le chloro-8-octanol-1, le cyclohexylméthanol, l'éthyl-2-hexanol-1, le but-3-én-ol-1, l'alcool propargylique, le 2-(4-méthoxyphényl)éthanol-1, le cyclohexanol.

Le procédé selon l'invention est particulièrement intéressant pour préparer les chlorures d'alcools acétyléniques et en particulier le chlorure de propargyle.

Le chlorure de thionyle ou, de préférence, le phosgène est mis à réagir avec l'alcool en proportion comprise entre 1 et 10 moles, de préférence entre 1 et environ 2 moles, par mole de groupe hydroxyle à transformer.

La réaction est en général effectuée à une température comprise entre 20° et 150°C, de préférence entre environ 50° et environ 130°C, et sous la pression atmosphérique. On peut également, si nécessaire, opérer sous une pression plus faible ou plus élevée, notamment comprise entre environ 0,1 bar et environ 20 bars.

Un solvant inerte vis à vis des composés et avec un point d'ébullition supérieur à la température de la réaction, peut être utilisé afin notamment de permettre une meilleure dissolution ou mise en suspension du catalyseur et un meilleur contact avec les réactifs.

Comme exemples de solvant, on peut citer les hydrocarbures aromatiques chlorés ou non tels que le toluène, le monochlorobenzène, les xylènes, les dichlorobenzènes, et les hydrocarbures aliphatiques.

Les catalyseurs nécessaires pour réaliser le procédé selon l'invention sont les sels de guanidinium hexasubstitués de formules (II) ou (III). Dans ces formules, A représente de préférence le chlore ou le groupe HCl₂.

Les substituants des radicaux R³ à R⁸ sont choisis parmi les groupes inertes dans les conditions de la réaction tels que les atomes d'halogène, les groupes alkyle, alkoxy, aryloxy et nitro.

Les radicaux R³ à R⁸, identiques ou différents, sont de préférence des groupes alkyles en C₁ à C₄. Le radical R'³ est de préférence le groupe -(CH₂)₃-.

Les supports de silice sont généralement des billes de silice qui contiennent des fonctions silanol en surface.

Ces sels de guanidinium, greffés ou non, se trouvent dans le commerce ou peuvent se préparer de manière connue, notamment comme décrit dans le brevet EP 545 774.

Comme exemples de catalyseurs de formule (II), on peut citer le chlorure ou le bromure d'hexaméthylguanidinium, le chlorure ou le bromure d'hexaéthylguanidinium, le chlorure ou le bromure d'hexabutylguanidinium, et leurs chloro ou bromohydrates.

Le chlorure d'hexabutylguanidinium ou son chlorhydrate sont les catalyseurs préférés.

Parmi les catalyseurs de formule (III) greffés sur un support à base de silice, on préfère le catalyseur dont les radicaux R⁴ à R⁸ sont des groupes butyle et R'³ représente le groupe -(CH₂)₃-.

La quantité de catalyseurs employée est généralement comprise entre 0,001 et 0,20 mole de groupe guanidinium par mole de groupe hydroxyle à transformer et de préférence entre environ 0,005 et environ 0,05 mole.

Les catalyseurs sont très stables dans les conditions réactionnelles. Ils ne perdent pas leur activité et peuvent facilement être réutilisés dans d'autres opérations.

Le procédé peut être réalisé en continu ou en discontinu.

Lorsqu'on opère en discontinu, on préfère ajouter progressivement le phosgène dans le milieu réactionnel qui contient l'alcool et le catalyseur et éventuellement un solvant.

Pour la préparation de certains chlorures, en particulier pour la préparation des chlorures acétyléniques, on préfère opérer en continu.

Ainsi, lorsque la réaction est effectuée à partir de l'alcool propargylique, celui-ci, liquide, et le phosgène, de préférence gazeux, sont introduits dans le milieu réactionnel contenant le catalyseur et éventuellement le solvant, chauffé à la température choisie, de préférence comprise entre 90°C et 130°C. Le chlorure de propargyle se forme rapidement. Celui-ci et les autres gaz qui se dégagent sont séparés du milieu réactionnel au fur et à mesure de leur formation, par exemple en les faisant passer dans une colonne à distiller. On recueille alors le chlorure de propargyle. Les analyses du distillat, effectuées par chromatographie en phase gazeuse, montrent qu'il ne contient pratiquement ni chloroformiate ni carbonate de propargyle.

Le procédé selon l'invention permet d'obtenir les chlorures aliphatiques ou cycloaliphatiques rapidement avec de bons rendements et peu de produits secondaires et notamment peu ou pas de chloroformiates et de carbonates. Pour la préparation du chlorure de propargyle, l'absence du carbonate de dipropargyle est particulièrement avantageuse car celui-ci, en raison de son instabilité et de sa toxicité, est un sous produit tout à fait indésirable.

Les exemples qui suivent illustrent l'invention sans toutefois la limiter.

### Exemple 1

Dans un ballon de 1 litre, muni d'une colonne de Vigreux surmontée d'un système de réfrigération à 0°C, on introduit, en pied de cuve, 300 g de xylène (mélange d'isomères), 25 g (environ 0,058 mol) de chlorure d'hexabutylguanidinium et 17 g (environ 0,17 mol) de phosgène.

Le milieu est porté à 100°C. On effectue alors une coulée simultanée d'alcool propargylique introduit liquide à l'aide d'une pompe péristaltique et de phosgène introduit gazeux à l'aide d'un tube plongeur.

Les quantités introduites en 2 heures sont respectivement de 163 g (environ 2,9 mol) d'alcool pour 398 g (environ 4,02 mol) de phosgène. Le milieu réactionnel est maintenu à 100°C.

Rapidement, après le début de la coulée simultanée, on observe une élévation de la température en tête de la colonne à distiller. Elle se stabilise vers 60-65°C.

La quantité de distillat recueilli est de 237,5 g. Sa composition, déterminée par chromatographie en phase gazeuse (CPG), est la suivante :

| | |
|---|---|
| Chlorure de propargyle | 57,6%. |
| Xylènes | 36,6%. |
| Alcool propargylique | 2,6%. |

On ne détecte ni la présence de carbonate de dipropargyle ni celle de chloroformiate de propargyle.

Le chlorure de propargyle est isolé par distillation sous pression atmosphérique à 60°C. Le rendement est de 60%.

### Exemple comparatif 1

On utilise le même mode opératoire qu'à l'exemple 1, mais à la place du chlorure d'hexabutylguanidinium, on introduit 0,058 mol de diméthylformamide, en pied de cuve.

La coulée simultanée dure deux heures pendant lesquelles on introduit 400 g de phosgène et 194 g d'alcool propargylique.

On recueille 250 g de distillat dont la composition déterminée par CPG est la suivante :

| | |
|---|---|
| chlorure de propargyle | 32% |
| alcool propargylique | 2,5% |
| xylènes | 54% |
| chloroformiate de propargyle | 11% |

Cet exemple montre qu'avec un catalyseur différent de ceux de l'invention, on obtient une quantité nettement inférieure de chlorure de propargyle et une quantité importante de chloroformiate de propargyle, comme sous produit.

### Exemple 2

Dans un réacteur de 100 l, muni d'une colonne à distiller surmontée d'un système réfrigérant, on introduit, en pied de cuve, 30 kg de xylènes et 2,5 kg de chlorhydrate de chlorure d'hexabutylguanidinium, puis, on ajoute 100 g de phosgène.

Le milieu est porté à 100°C. On effectue alors une coulée simultanée d'alcool propargylique et de phosgène. L'alcool est introduit à l'aide d'une pompe doseuse à un débit variant de 1,5 à 3 kg/h et le phosgène est introduit sous forme gazeuse, à l'aide d'un tube plongeur à un débit variant de 3 à 7 kg/h, de façon à ce que le milieu réactionnel soit toujours en excès de phosgène. La température du milieu réactionnel est maintenue entre 98°C et 108°C.

En 15 heures, on introduit ainsi 74,4 kg de phosgène et 29,2 kg d'alcool propargylique.

On recueille 31,9 kg de distillat, essentiellement constitué de chlorure de propargyle contenant 4 % de phosgène résiduel ainsi que 3 % d'autres impuretés (déterminé par analyse CPG). Le rendement brut est de 80%.

### Exemple 3

Dans un ballon tricol de 250 ml, muni d'une colonne à distiller surmontée d'un système réfrigérant, on introduit en pied de cuve, 60 g de xylène et 5 g de chlorhydrate de chlorure d'hexabutylguanidinium, (1% en mol par rapport au butanol).

Le milieu est porté à une température comprise entre 120 et 130°C. On introduit alors environ 5 g de phosgène. Puis on effectue une coulée simultanée de n-butanol et de phosgène de façon à ce que le milieu soit toujours en excès de phosgène. On introduit ainsi environ 80 g de n-butanol et 155 g de phosgène (excès de 50%), en maintenant le milieu réactionnel à 120-130°C, en 8 heures.

La quantité de distillat recueilli pendant la coulée simultanée est de 46 g. Il est composé de 98% (déterminée par analyse CPG) de chlorure de n-butyle.

## Revendications

1. Procédé de préparation des chlorures d'hydrocarbures aliphatiques ou cycloaliphatiques, saturés ou non, substitués ou non, par réaction du mono- ou du polyalcool correspondant avec le phosgène ou le chlorure de thionyle, **caractérisé en ce que** la réaction est effectuée en présence d'au moins un catalyseur choisi dans le groupe constitué par :
- (1) les halogénures de guanidinium hexasubstitués et leurs halogénohydrates de formule (II) : 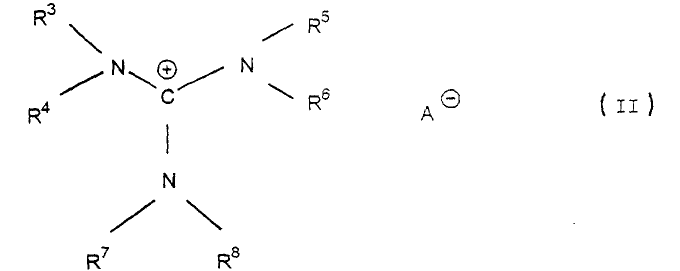 dans laquelle les radicaux R³ à R⁸, identiques ou différents, représentent des groupes alkyles en C₁ à C₁₂ linéaires ou ramifiés, substitués ou non, et/ou cycloalkyles en C₅ à C₆, substitués ou non, et A représente un atome de chlore ou de brome ou un groupe HCl₂ ou HBr₂, et
- (2) les halogénures de guanidinium hexasubstitués et leurs halogénohydrates, greffés par l'intermédiaire d'un de leurs radicaux sur un support à base de silice, de formule (III) : 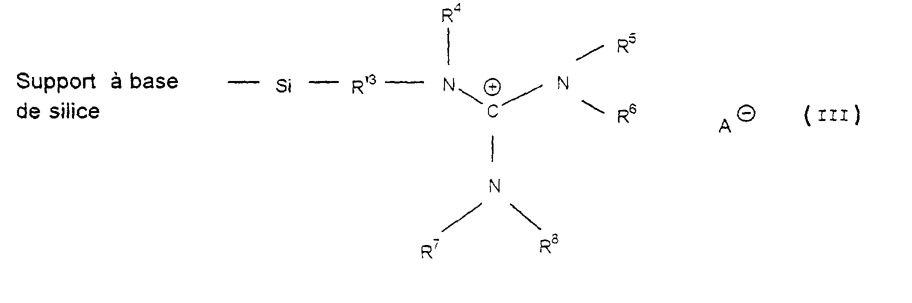 dans laquelle les radicaux R⁴ à R⁸ et A ont les significations précédentes et R'³ représente un groupe alkylène en C₂ à C₁₀.

2. Procédé de préparation selon la revendication 1, **caractérisé en ce que** l'alcool est représenté par la formule (I) : 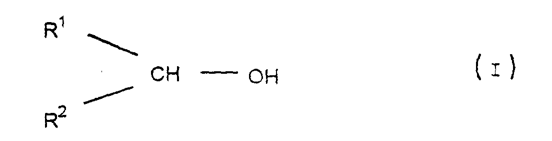 dans laquelle R¹ et R², identiques ou différents, représentent un atome d'hydrogène, un groupe aliphatique en C₁ à C₃₀, linéaire ou ramifié, substitué ou non, saturé ou non, un groupe cycloaliphatique en C₃ à C₁₂, substitué ou non, saturé ou non, un groupe alcoxy en C₁ à C₃₀, substitué ou non, saturé ou non, ou un groupe aryle ou aryloxy, substitué ou non;
ou bien dans laquelle R¹ et R² forment ensemble et avec le carbone sur lequel ils sont fixés, un groupe cycloaliphatique en C₃ à C₁₂, substitué ou non, saturé ou non.

3. Procédé selon la revendication 2, **caractérisé en ce que** les substituants des radicaux R¹ et R² sont choisis parmi les atomes d'halogène, les groupes hydroxyles, aliphatiques saturés ou non, substitués ou non, halogénoaliphatiques, cycloaliphatiques saturés ou non, substitués ou non, aryles substitués ou non, araliphatiques saturés ou non, substitués ou non, alcoxy, polyalcoxy, alcoxyaryles, aryloxy, nitro et cyano.

4. Procédé selon la revendication 1, **caractérisé en ce que** A représente le chlore ou le groupe HCl₂.

5. Procédé selon la revendication 1, **caractérisé en ce que** les radicaux R³ à R⁸, identiques ou différents, représentent des groupes alkyles en C₁ à C₄ et le radical R'³ représente le groupe -(CH₂)₃-.

6. Procédé selon l'une quelconque des revendications précédentes, **caractérisé en ce que** la quantité de catalyseur utilisée est comprise entre 0,001 et 0,20 mole de groupe guanidinium par mole de groupe hydroxyle à transformer.

7. Procédé selon l'une quelconque des revendications précédentes, **caractérisé en ce que** la quantité de phosgène est comprise entre 1 et 10 moles par mole de groupe hydroxyle à transformer.

8. Procédé selon l'une quelconque des revendications précédentes, **caractérisé en ce que** la température est comprise entre 20° et 150°C.

9. Procédé selon l'une quelconque des revendications précédentes, **caractérisé en ce que** la réaction est effectuée dans un milieu solvant inerte vis à vis des composés, de point d'ébullition supérieur à la température de la réaction.

10. Procédé selon l'une quelconque des revendications précédentes, **caractérisé en ce que** le procédé est réalisé en continu.

11. Procédé selon la revendication 2 ou 10, **caractérisé en ce que** l'alcool est l'alcool propargylique.

12. Procédé selon la revendication 11, **caractérisé en ce que** la température de réaction est comprise entre 90° et 130°C.

## Patentansprüche

1. Verfahren zur Herstellung von Chloriden von aliphatischen oder cycloaliphatischen, gesättigten oder ungesättigten, substituierten oder unsubstituierten Kohlenwasserstoffen durch Umsetzung eines entsprechenden Mono- oder Polyalkohols mit Phosgen oder Thionylchlorid, **dadurch gekennzeichnet, daß** die Umsetzung in Gegenwart mindestens eines Katalysators durchgeführt wird, der ausgewählt ist unter:
- (1) sechsfach substituierten Guanidiniumhalogeniden und den Hydrohalogeniden dieser Verbindungen der Formel (II): 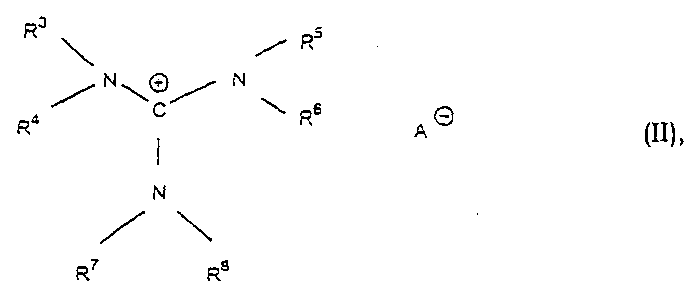 worin die Gruppen R³ bis R⁸, die identisch oder voneinander verschieden sind, geradkettige oder verzweigte, substituierte oder unsubstituierte C₁₋₁₂-Alkylgruppen und/oder substituierte oder unsubstituierte C₅₋₆-Cycloalkylgruppen bedeuten und A ein Chlor- oder Bromatom oder eine Gruppe HCl₂ oder HBr₂ ist, und
- (2) sechsfach substituierten Guanidiniumhalogeniden und den Hydrohalogeniden dieser Verbindungen der Formel (III), die über eine ihrer Gruppen auf einen Träger auf der Basis von Siliciumdioxid gepfropft sind: 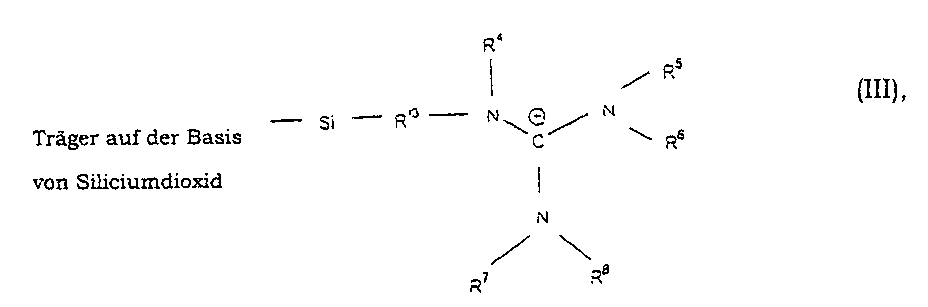 worin die Gruppen R⁴ bis R⁸ und A die oben angegebenen Bedeutungen aufweisen und R'³ eine C₂₋₁₀-Alkylengruppe bedeutet.

2. Verfahren zur Herstellung nach Anspruch 1, **dadurch gekennzeichnet, daß** der Alkohol der Formel (I) entspricht: 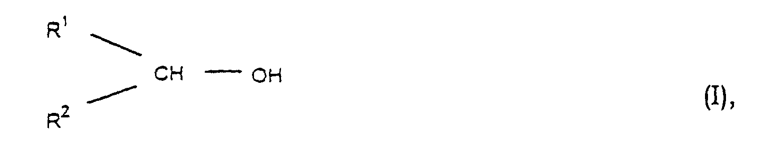 worin die Gruppen R¹ und R², die identisch oder voneinander verschieden sind, Wasserstoff, eine gesättigte oder ungesättigte, substituierte oder unsubstituierte, geradkettige oder verzweigte aliphatische Gruppe mit 1 bis 30 Kohlenstoffatomen, eine gesättigte oder ungesättigte, substituierte oder unsubstituierte cycloaliphatische Gruppe mit 3 bis 12 Kohlenstoffatomen, eine gesättigte oder ungesättigte, substituierte oder unsubstituierte Alkoxygruppe mit 1 bis 30 Kohlenstoffatomen oder eine substituierte oder unsubstituierte Aryl-oder Aryloxygruppe bedeuten;
oder worin R¹ und R² gemeinsam mit dem Kohlenstoffatom, an das sie gebunden sind, eine gesättigte oder ungesättigte, substituierte oder unsubstituierte cycloaliphatische Gruppe mit 3 bis 12 Kohlenstoffatomen bilden.

3. Verfahren nach Anspruch 2, **dadurch gekennzeichnet, daß** die Substituenten der Gruppen R¹ und R² unter den Halogenatomen, Hydroxygruppen, gesättigten oder ungesättigten, substituierten oder unsubstituierten aliphatischen Gruppen, halogenaliphatischen Gruppen, gesättigten oder ungesättigten, substituierten oder unsubstituierten cycloaliphatischen Gruppen, substituierten oder unsubstituierten Arylgruppen, gesättigten oder ungesättigten, substituierten oder unsubstituierten araliphatischen Gruppen, Alkoxygruppen, Polyalkoxygruppen, Alkoxyarylgruppen, Aryloxygruppen, Nitrogruppen und Cyanogruppen ausgewählt sind.

4. Verfahren nach Anspruch 1, **dadurch gekennzeichnet, daß** A Chlor oder HCl₂ bedeutet.

5. Verfahren nach Anspruch 1, **dadurch gekennzeichnet, daß** die Gruppen R³ bis R⁸, die identisch oder voneinander verschieden sind, C₁₋₄-Alkylgruppen bedeuten und die Gruppe R'³ die Gruppe -(CH₂)₃- ist.

6. Verfahren nach einem der vorhergehenden Ansprüche, **dadurch gekennzeichnet, daß** die verwendete Katalysatormenge im Bereich von 0,001 bis 0,20 mol der Guanidiniumgruppe pro Mol der umzuwandelnden Hydroxygruppe liegt.

7. Verfahren nach einem der vorhergehenden Ansprüche, **dadurch gekennzeichnet, daß** die Phosgenmenge im Bereich von 1 bis 10 mol pro Mol der umzuwandelnden Hydroxygruppe liegt.

8. Verfahren nach einem der vorhergehenden Ansprüche, **dadurch gekennzeichnet, daß** die Temperatur im Bereich von 20 bis 150 °C liegt.

9. Verfahren nach einem der vorhergehenden Ansprüche, **dadurch gekennzeichnet, daß** die Umsetzung in einem gegenüber den Verbindungen inerten Lösungsmittelmedium mit einem Siedepunkt über der Umsetzungstemperatur durchgeführt wird.

10. Verfahren nach einem der vorhergehenden Ansprüche, **dadurch gekennzeichnet, daß** das Verfahren kontinuierlich durchgeführt wird.

11. Verfahren nach Anspruch 2 oder 10, **dadurch gekennzeichnet, daß** es sich bei dem Alkohol um Propargylalkohol handelt.

12. Verfahren nach Anspruch 11, **dadurch gekennzeichnet, daß** die Reaktionstemperatur im Bereich von 90 bis 130 °C liegt.

## Claims

1. Method for preparing the chlorides of substituted or unsubstituted, saturated or unsaturated aliphatic or cycloaliphatic hydrocarbons, by reacting the corresponding mono- or polyalcohol with phosgene or thionyl chloride, **characterized in that** the reaction is carried out in the presence of at least one catalyst chosen from the group consisting of:
- (1) hexa-substituted guanidinium halides and their hydrohalides of formula (II): 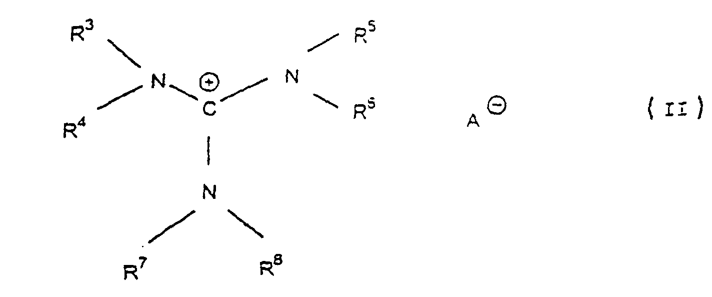 in which the radicals R³ to R⁸, whether identical or different, represent substituted or unsubstituted, linear or branched alkyl groups with C₁ to C₁₂ and/or substituted or unsubstituted cycloalkyls with C₅ to C₆, and A represents a chlorine or bromine atom or an HCl₂ or HBr₂ group, and
- (2) hexa-substituted guanidinium halides and their hydrohalides, grafted by means of one of their radicals onto a silica-based support, of formula (III): 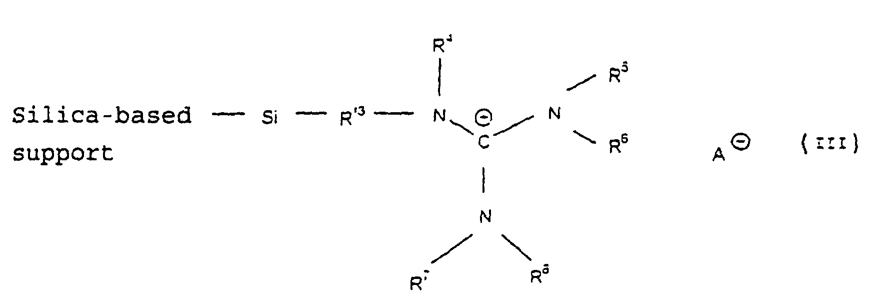 in which the radicals R⁴ to R⁸ and A have the preceding meanings and R'³ represents an alkalene group with C₂ to C₁₀.

2. Preparative method according to claim 1, **characterized in that** the alcohol is represented by the formula (I): 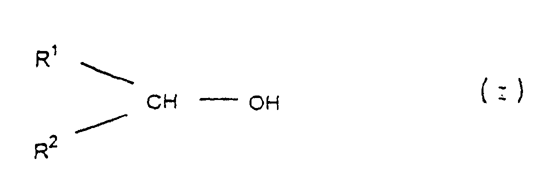 in which R¹ and R², whether identical or different, represent a hydrogen atom, a saturated or unsaturated, substituted or unsubstituted, linear or branched aliphatic group with C₁ to C₃₀, a saturated or unsaturated, substituted or unsubstituted cycloaliphatic group with C₃ to C₁₂, or a substituted or unsubstituted aryl or aryloxy group;
or else in which R¹ and R² form together and with the carbon to which they are attached, a saturated or unsaturated, substituted or unsubstituted cycloaliphatic group with C₃ to C₁₂.

3. Method according to claim 2, **characterized in that** the substituents of the radicals R¹ and R² are chosen from halogen atoms, hydroxyl groups, substituted or unsubstituted, saturated or unsaturated aliphatic groups, halegenoaliphatic groups, substituted or unsubstituted, saturated or unsaturated cycloaliphatic groups, substituted or unsubstituted aryl groups, substituted or unsubstituted saturated or unsaturated araliphatic groups, alkoxy groups, polyalkoxy groups, alkoxyaryl groups, aryloxy groups, nitro groups and cyano groups.

4. Method according to claim 1, **characterized in that** A represents chlorine or the HCl₂ group.

5. Method according to claim 1, **characterized in that** the radicals R³ to R⁸, whether identical or different, represent alkyl groups with C₁ to C₄ and the radical R'³ represents the -(CH₂)₃- group.

6. Method according to any one of the preceding claims, **characterized in that** the quantity of catalyst used lies between 0.001 and 0.20 mole of the guanidinium group per mole of the hydroxyl group to be converted.

7. Method according to any one of the preceding claims, **characterized in that** the quantity of phosgene lies between 1 and 10 mole per mole of the hydroxyl group to be converted.

8. Method according to any one of the preceding claims, **characterized in that** the temperature lies between 20°C and 150°C.

9. Method according to any one of the preceding claims, **characterized in that** the reaction is carried out in a solvent medium which is inert to the compounds and has a boiling point above the reaction temperature.

10. Method according to any one of the preceding claims, **characterized in that** the method is carried out continuously.

11. Method according to claim 2 or 10, **characterized in that** the alcohol is propargylic alcohol.

12. Method according to claim 11, **characterized in that** the reaction temperature lies between 90° and 130°C.
